## Europäisches Patentamt

## European Patent Office

## Office européen des brevets

(19)

(11) EP 0 943 312 A2

(12) **EUROPÄISCHE PATENTANMELDUNG**

(43) Veröffentlichungstag:
22.09.1999 Patentblatt 1999/38

(51) Int. Cl.⁶: **A61K 7/06**

(21) Anmeldenummer: 99100373.2

(22) Anmeldetag: 15.01.1999

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 11.02.1998 DE 19805434

(71) Anmelder:
Wella Aktiengesellschaft
64274 Darmstadt (DE)

(72) Erfinder:
• Karlen, Thomas, Dr.
3013 Bern (CH)
• Chambettaz, Daniel
1717 St. Ursen (CH)

(54) **Haarbehandlungsmittel mit fluorierten Säuren und Polymeren**

(57)    Es wird die Verwendung von fluorierten Säuren zur Neutralisation von Polymeren mit basischen Gruppen in Mitteln zur Behandlung von Haaren sowie ein Haarbehandlungsmittel beschrieben, welches eine Mischung enthält aus (A) einem Komplex gebildet aus mindestens einer fluorierten Säure und mindestens einem Polymeren mit basischen Gruppen bzw. einem Komplex eines Salzes einer fluorierten Säure und einem Polymeren mit protonierten oder quaternären Amingruppen und (B) mindestens einem filmbildenden, haarfestigenden Polymeren.

Das erfindungsgemäße Mittel bewirkt eine Verringerung der Rückstandsbildung auf dem Haar, ohne die Festigungsleistung zu beeinträchtigen.

**Beschreibung**

Gegenstand der Erfindung ist die Verwendung von fluorierten Säuren zur Neutralisation von Polymeren mit basischen Gruppen in Mitteln zur Behandlung von Haaren. Ein weiterer Gegenstand der Erfindung ist ein Haarbehandlungsmittel mit einem Gehalt an (A) einem Komplex gebildet aus mindestens einer fluorierten Säure und mindestens einem Polymeren mit basischen Gruppen bzw. einem Komplex eines Salzes einer fluorierten Säure und einem Polymeren mit protonierten oder quaternären Amingruppen und (B) mindestens einem filmbildenden, haarfestigenden Polymeren.

Haarstylingprodukte sind solche Produkte, welche den Haaren durch Polymerzusätze Halt, Volumen, Elastizität, Sprungkraft und Glanz geben. Sie dienen in erster Linie dazu, die Haare in Form zu bringen (zum Beispiel Gele), dem Haar Stand zu verleihen (zum Beispiel Haarsprays) und dem Haar Volumen zu geben (zum Beispiel Festigerschäume).

Bei Stylingprodukten ist es ein bekanntes Problem, daß Rückstände in Form von feinen oder groben Schuppen entstehen können, wenn das Haar durch Bewegung gegeneinander verschoben wird. Dies geschieht z.B. beim Kämmen oder Bürsten der Haare oder auch beim Kontakt des Kopfs mit Stoffen wie z.B. bei Bewegungen des Kopfs auf Kopfkissen. Solche Rückstände werden vom Verbraucher als unästhetisch empfunden und machen eine häufige Haarwäsche nötig. Es ist deshalb erstrebenswert, das Rückstandsverhalten von Stylingprodukten möglichst zu minimieren, ohne dabei die positiven Eigenschaften des Produkts zu beeinträchtigen.

Es ist bekannt, das Rückstandsverhalten von Haarfestigungsmitteln dadurch zu verbessern, daß Stoffe wie z.B. Siliconöle, tensidische Verbindungen, Öle u.a. in die Rezeptur eingearbeitet werden. Die Verwendung dieser Stoffe hat allerdings den Nachteil, daß die Filme weicher werden, was eine Verringerung der Festigungsleistung mit sich zieht. Zur Erzielung eines bestimmten Frisurenhalts muß somit entweder eine größere Produktmenge appliziert werden, oder der Polymergehalt im Produkt muß gesteigert werden. Die Zusatzstoffe haben zudem den Nachteil, daß sie das Haar belasten und ihm einen öligen, fettigen Aspekt verleihen. Es bestand somit die Aufgabe, ein Mittel zu finden, welches ein gutes Rückstandsverhalten aufweist, ohne die oben erwähnten Nachteile zu besitzen.

Es wurde nun gefunden, daß die Verwendung von fluorierten Säuren in Stylingprodukten, welche mindestens ein Polymer mit basischen Gruppen enthalten, eine Verbesserung des Rückstandsverhaltens bewirkt, ohne daß eine Verschlechterung der Festigung oder eine Belastung der Haare resultiert.

Gegenstand der Erfindung ist die Verwendung von fluorierten Säuren zur Neutralisation von Polymeren mit basischen Gruppen in Mitteln zur Behandlung von Haaren, insbesondere in Mitteln zur Festigung von Haaren. Polymere mit basischen Gruppen im Sinne der Erfindung sind Homo- oder Copolymere, die aus mindestens einer Monomerart aufgebaut sind, die eine basische Gruppe, insbesondere ein basisches Stickstoffatom enthält. Vorzugsweise handelt es sich um Polymere, die außer den basischen Gruppen keine sauren oder anionischen Gruppen enthalten, also keinen amphoteren Charakter haben.

Ein weiterer Gegenstand der Erfindung ist ein Haarbehandlungsmittel mit einem Gehalt an einem Komplex gebildet aus mindestens einer fluorierten Säure und mindestens einem Polymeren mit primären oder sekundären Amingruppen bzw. einem Komplex eines Salzes einer fluorierten Säure und einem Polymeren mit protonierten primären oder sekundären Amingruppen. Polymere mit primären oder sekundären Amingruppen im Sinne der Erfindung sind Homo- oder Copolymere, die aus mindestens einer Monomerart aufgebaut sind, die mindestens eine primäre oder sekundäre Aminguppe enthält. Auch bei diesen polymeren handelt es sich vorzugsweise um Polymere, die außer den basischen Gruppen keine sauren oder anionischen Gruppen enthalten, also keinen amphoteren Charakter haben.

Ein weiterer Gegenstand der Erfindung ist ein Haarbehandlungsmittel mit einem Gehalt an

(A) einem Komplex gebildet aus mindestens einer fluorierten Säure und mindestens einem Polymeren mit basischen Gruppen bzw. einem Komplex eines Salzes einer fluorierten Säure und einem Polymeren mit protonierten oder quaternären Amingruppen, wobei das Polymer vorzugsweise frei von sauren oder anionischen Gruppen ist und
(B) zusätzlich mindestens einem filmbildenden, haarfestigenden Polymeren.

Das filmbildende, haarfestigende Polymer (B) liegt vorzugsweise in einer Menge von 0,01 bis 50 Gewichtsprozent, besonders bevorzugt in einer Menge von 0,5 bis 50 Gewichtsprozent in dem erfindungsgemäßen Mittel vor.

Unter dem Begriff „Komplex" im Sinne der vorliegenden Erfindung wird ein Ionenpaar verstanden, welches aus mindestens einem Anion einer fluorierten Säure und einem Polymeren mit kationischen Gruppen gebildet wird.

Die fluorierte Säure bzw. dessen Salz liegt vorzugsweise in einer Menge von 0,01 bis 10 Gewichtsprozent, besonders bevorzugt von 0,05 bis 5 Gewichtsprozent vor. Das Polymer mit basischen Gruppen bzw. das Polymer mit protonierten Amingruppen liegt vorzugweise in einer Menge von 0,01 bis 20 Gewichtsprozent vor. Es werden wässrige oder wässrig-alkoholische Lösungsmittelsysteme bevorzugt.

Vorzugsweise ist die fluorierte Säure eine Verbindung mit der allgemeinen Formel (I)

$$A\text{-}(CH_2)_x\text{-}(CF_2)_y\text{-}(CH_2)_z\text{-}B \hspace{6cm} (I)$$

wobei x und z unabhängig voneinander Zahlen von 0 bis 5 bedeuten, y eine Zahl von 1 bis 21 bedeutet, A eine Säuregruppe ist und B entweder für eine Säuregruppe oder für ein Fluoratom steht, wobei z den Wert 0 hat, wenn B für ein Fluoratom steht. Die Säuregruppen A- und B- sind vorzugsweise ausgewählt aus -COOH, -SO$_3$H, -OPO$_2$H und -OPO$_3$H$_2$.

Geeignete fluorierte Säuren sind insbesondere perfluorierte Säuren wie Trifluormethansulfonsäure, Perfluorpropionsäure, Perfluorbuttersäure, Perfluorpentansäure, Perfluorhexansäure, Perfluorheptansäure, Perfluoroctansäure, Perfluornonansäure, Perfluordecansäure, Perfluordodecansäure, Perfluorglutarsäure, Perfluorsebacinsäure, Perfluoroctansulfonsäure, die von der Firma Clariant (Deutschland) unter der Handelsbezeichnung Fluowet$^{®}$ PL 80 vertriebene Mischung aus perfluorierten Phosphin-/Phosphonsäuren oder die von der Firma 3M unter der Handelsbezeichnung Fluorad$^{®}$ FX-1001 vertriebene Perfluoroctansäure.

Weitere geeignete fluorierte Säuren sind teilfluorierte Säuren wie beispielsweise 3H-Decafluorhexansäure, 6-Fluorhexansäure, 4,4,5,5,6,6,6-Heptafluorhexansäure, 5,5,5-Trifluorvaleriansäure, 2H,2H-Heptafluorvalerian-säure, 5H-Octafluorvaleriansäure, 3H- Octafluorvaleriansäure oder 2H-Decafluor-pentan-1-sulfonsäure.

Bevorzugt sind solche Verbindungen gemäß Formel (I), welche in wässriger oder in wässrig/ethanolischer Lösung bei einem Wassergehalt von mindestens 10% löslich sind. Besonders bevorzugt sind solche Verbindungen gemäß Formel (I), welche in rein wässriger Lösung löslich sind, wie z.B. Perfluorpentansäure, Perfluorbuttersäure, Perfluorpropionsäure oder Perfluorglutarsäure.

Die eingesetzten Polymere mit basischen Gruppen haben ein Molekulargewicht von vorzugsweise mindestens 50.000 g/mol, besonders bevorzugt von 100.000 bis 6.000.000 g/mol und enthalten stickstoffhaltige Gruppen wie zum Beispiel primäre, sekundäre oder tertiäre Amine. Der Vorteil von Polymeren mit primären oder sekundären Amingruppen besteht in einer großen Affinität der fluorierten Säure zu diesen Gruppen. Der daraus resultierende Komplex zwischen Polymer und Fluorsäure ist dadurch stabilisiert, die Fluorreste verbleiben im Polymerfilm und migrieren nicht aus ihm heraus.

Die basische Gruppe ist entweder in der polymerkette oder vorzugsweise als Substituent an einem oder mehreren Monomeren enthalten. Das Polymer mit basischen Gruppen kann ein natürliches oder ein synthetisches Homo- oder Copolymer mit aminsubstituierten Monomereinheiten sowie gegebenenfalls mit nicht-basischen Comonomeren sein. Geeignete polymere mit basischen Gruppen sind zum Beispiel Copolymere von aminsubstituierten Vinylmonomeren und nicht aminsubstituierten Monomeren. Aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise niedere Alkylgruppen wie zum Beispiel C1- bis C7-Alkylgruppen, besonders bevorzugt C1- bis C3-Alkylgruppen sind.

Nicht aminsubstituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Maleinsäureanhydrid, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Ein bevorzugt eingesetztes Polymer mit basischen Gruppen ist Chitosan oder ein Chitosanderivat, insbesondere Chitosan mit einem Molekulargewicht von 20.000 bis ca. 5 Millionen g/mol. Geeignet ist beispielsweise ein niedermolekulares Chitosan mit einem Molekulargewicht von 30.000 bis 70.000 g/mol oder ein hochmolekulares Chitosan mit einem Molekulargewicht von 300.000 bis 700.000 g/mol. Der bevorzugte Entacetylierungsgrad des Chitosans liegt zwischen 10 und 99 Prozent. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen.

Weitere, vorzugsweise eingesetzte Polymere mit basischen Gruppen sind amphotere polymere wie zum Beispiel Copolymere gebildet aus Alkylacrylamid, insbesondere Octylacrylamid, Alkylaminoalkylmethacrylat, insbesondere t-Butylaminoethylmethacrylat und zwei oder mehr Monomeren bestehend aus Acrylsäure, Methacrylsäure oder deren Ester, wie sie zum Beispiel unter den Handelsnamen Resyn 28-4910, Amphomer oder Amphomer LV-71 der Firma National Starch, USA erhältlich sind.

Die basischen Gruppen des Polymers sind im erfindungsgemässen Mittel teilweise oder vollständig mit der fluorierten Säure neutralisiert. Bevorzugt sind jedoch solche Mittel, in welchen die basischen Gruppen des Polymers zu 5 bis 70% mit der fluorierten Säure neutralisiert sind und eine weitere Neutralisation durch zusätzliche, nicht fluorierte Neutralisationsmittel, welche in der Haarkosmetik üblicherweise eingesetzt werden, vorgenommen werden kann. Als zusätzliche Neutralisationsmittel können organische oder anorganische Säuren verwendet werden. Beispiele für derartige Säuren sind Ameisensäure, Weinsäure, Äpfelsäure, Milchsäure, Zitronensäure, Pyrrolidon-carbonsäure, Salzsäure u.a..

Besitzt das Polymer mit basischen Gruppen zusatzlich säurehaltige Gruppen, wie dies bei amphoteren Polymeren der Fall sein kann, so können die sauren Gruppen mit einer Base neutralisiert werden, bevor die basischen Gruppen mit der fluorieren Säure neutralisiert werden. Beispiele für geeignete Basen sind Aminomethylpropanol, Triethanolamin, Monoethanolamin, Natronlauge u.a..

Die Polymere mit basischen Gruppen können wasserlöslich oder wasserunlöslich sein. Bevorzugt sind jedoch Polymere, welche wasserunlöslich sind, wenn die basischen Gruppen in unneutralisierter Form vorliegen und erst nach teilweiser oder vollständiger Neutralisation mit den fluorierten Säuren wasserlöslich sind.

Die Polymere mit basischen Gruppen können substantiv oder nicht substantiv zu keratinischem Material sein. Bevorzugt sind Polymere, welche nach Neutralisation nicht substantiv sind. Unter dem Begriff „nicht substantiv" sind im allgemeinen jene Polymere gemeint, die durch einmaliges Waschen der Haare wieder entfernt werden können oder auch bereits durch einfaches Spülen mit Wasser aus dem Haar entfernt werden.

Die Polymere mit quaternären Amingruppen, welche in dem erfindungsgemäßen Mittel in Kombination mit Salzen von fluorierten Säuren und mindestens einem filmbildenden, haarfestigenden Polymeren eingesetzt werden, können Homo- oder Copolymere sein, wobei die quaternären Stickstoffgruppen entweder in der Polymerkette oder vorzugsweise als Substituent an einem oder mehreren Monomeren enthalten sind. Die Ammoniumgruppen enthaltenden Monomere können mit den oben genannten nicht aminsubstituierten Monomeren copolymerisiert sein. Geeignete ammoniumsubstituierte Vinylmonomere sind zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazolium-chlorid/Vinylpyrrolidon Copolymer (Polyquaternium-16), quaternisiertes Vinylpyrrolidon/Dimethylaminoethylmeth-acrylat Copolymer (Polyquaternium-11), Homo- und Copolymere von Dimethyldiallylammoniumchlorid (Polyquaternium-6 und -7), quaternisierte Hydroxyethylcellulose (Polyquaternium-10) oder quaternisierte Guarderivate.

Bei dem filmbildenden und haarfestigenden Polymer der Komponente (B) kann es sich um ein synthetisches oder natürliches, nichtionisches, kationisches, anionisches oder amphoteres Polymer handeln. Die haarfestigenden Polymere können einzeln oder in einem Gemisch eingesetzt werden. Unter filmbildenden, haarfestigenden Polymeren sollen solche Polymere verstanden werden, welche allein in 0,1 bis 5%iger wässriger, alkoholischer oder wässrig-alkoholischer Lösung angewandt, in der Lage sind, auf dem Haar einen Polymerfilm abzuscheiden und auf diese Weise das Haar zu festigen.

Geeignete synthetische, nichtionische, filmbildende, haarfestigende Polymere sind zum Beispiel Homopolymere des Vinylpyrrolidons, die beispielsweise unter den Handelsbezeichnungen LUVISKOL® K von der Firma BASF, Deutschland oder PVP-K von der Firma ISP, USA vertrieben werden, sowie Homopolymere des N-Vinylformamids, die beispielsweise unter der Handelsbezeichnung PVF von der Firma National Starch/USA vertrieben werden. Weitere geeignete synthetische filmbildende, nichtionische haarfestigende Polymere sind zum Beispiel Copolymerisate aus Vinylpyrrolidon und Vinylacetat, die beispielsweise unter den Handelsbezeichnungen LUVISKOL® VA von der Firma BASF/ Deutschland vertrieben werden; Terpolymere aus Vinylpyrrolidon, Vinylacetat und Vinylpropionat, die beispielsweise unter der Handelsbezeichnung LUVISKOL® VAP der Firma BASF/Deutschland vertrieben werden; Polyacrylamide, die beispielsweise unter den Handelsbezeichnungen AKYPOMINE® P 191 von der Firma CHEM-Y/ Deutschland oder SEPIGEL® 305 von der Firma SEPPIC/USA vertrieben werden; Polyvinylalkohole, die beispielsweise unter den Handelsbezeichnungen ELVANOl® der Firma Du Pont oder VINOL® 523/540 der Firma Air Porducts/USA vertrieben werden, sowie hochmolekulares Polyethylenglykol oder hochmolekulare Copolymere von Ethylenglykol mit Propylenglykol mit festigenden Eigenschaften, die beispielsweise unter den Handelsbezeichnungen LIPOXOL® 1000 von der Firma HÜLS AG/Deutschland, PLURACOL E 4000 von der Firma BASF/Deutschland oder UPIWAX® 20.000 von der Firma UPI vertrieben werden.

Geeignete synthetische filmbildende anionische Polymere sind zum Beispiel vernetzte oder unvernetzte Vinylacetat/Crotonsäure Copolymere, die beispielsweise in Form einer 60%igen Lösung in Isopropanol/Wasser unter der Handelsbezeichnung ARISTOFLEX® von der Firma HOECHST/Deutschland beziehungsweise von der Firma BASF unter dem Handelsnamen LUVISET CA-66 vertrieben werden. Weitere geeignete anionische Polymere sind zum Beispiel Terpolymere aus Acrylsäure, Alkylacrylat und N-Alkylacrylamid, insbesondere Acrylsäure/Ethylacrylat/N-t-Butylacrylamid Terpolymere, wie sie unter den Handelsnamen ULTRAHOLD 8 und ULTRAHOLD STRONG der Firma BASF/Deutschland vertrieben werden oder Terpolymere aus Vinylacetat, Crotonat und Vinylalkanoat, insbesondere Vinylacetat/Crotonat/Vinylneodecanoat Copolymere, wie sie z.B. von der Forma National Starch unter der Handelsbezeichnung RESYN 28-2930 vertrieben werden.

Geeignete natürliche filmbildende Polymere mit haarfestigender Wirkung sind zum Beispiel verschiedene Saccharidtypen wie zum Beispiel Polysaccharide oder Gemische aus Oligo-, Mono- und Disacchariden, welche beispielsweise unter dem Handelsnamen C-PUR® von der Firma Cerestar, Brüssel/Belgien vertrieben werden. Weitere geeignete, natürliche Polymere sind chinesiches Balsamharz und Cellulosederivate, zum Beispiel Hydroxy-propylcellulose mit einem Molekulargewicht von 30.000 bis 50.000 g/mol, welche beispielsweise unter der Handelsbezeichnung NISSO SL® von der Firma Lehmann & Voss/Deutschland vertrieben wird. Ein weiteres natürliches Polymer ist Schellack. Schellack kann in neutralisierter Form und unneutralisiert zum Einsatz kommen.

Von den kationischen Polymeren, die in dem erfindungsgemäßen Mittel enthalten sein können, ist zum Beispiel Polyvinylpyrrolidon/Dimethylaminoethylmethacrylat Copolymer, das unter der Handelsbezeichnung Gafquat 755 N von der Firma Gaf Co./USA vertrieben wird, geeignet. Weitere kationische Polymere sind beispielsweise das von der Firma BASF AG/Deutschland unter dem Handelsnamen LUVIQUAT® HM 550 vertriebene Copolymer aus Polyvinylpyrolidon und Imidazoliminmethochlorid, das von der Firma Calgon/USA unter dem Handelsnamen Merquat® Plus 3300 vertriebene Terpolymer aus Dimethyl-diallylammoniumchlorid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam, das von der Firma Amerchol/USA, unter dem Handelsnamen Polymer IR® vertriebene quaternierte Ammoniumsalz der Hydroxyethylcellulose und einem trimethylammonium-substituierten Epoxid, das von der Firma Gaf unter dem Handelsnamen Gafquat® HS 100 vertriebene Vinylpyrrolidon/Methacrylamidopropyltrimethylammoniumchlorid Copolymer und das von der Firma GOLDSCHMIDT/Deutschland unter dem Handelsnamen Abil® Quat 3272 vertriebene diquaternäre Polydimethylsiloxan.

Desweiteren können in dem erfindungsgemäßen Mittel Polymere mit verdickender Wirkung eingesetzt werden.

Von den Verdickern, die in dem erfindungsgemäßen Mittel enthalten sein können, sind Homopolymere der Acrylsäure mit einem Molekulargewicht von 2.000.000 bis 6.000.000 zu nennen, die beispielsweise von der Firma BF Goodrich/USA unter der Handelsbezeichnung Carbopol® vertrieben werden. Als weitere Verdicker kann das erfindungsgemäße Mittel ein Acrylsäurehomopolymer mit einem Molekulargewicht von 4.000.000 enthalten, das beispielsweise von der Firma BF Goodrich unter der Handelsbezeichnung Carbopol® 940 vertrieben wird. Weitere Verdicker sind beispielsweise die von der Firma BF Goodrich unter dem Handelsnamen Carbopol® ETD 2001 oder von der Firma Protex/Frankreich unter dem Handelsnamen Modarez V 600 PX vertriebene Acrylsäure-homopolymer, das von der Firma Hoechst/ Deutschland unter dem Handelsnamen Hostacerin® PN 73 vertriebene Polymer aus Acrylsäure und Acrylamid (Natriumsalz) mit einem Molekulargwicht von 2.000.000 bis 6.000.000 und das von der Firma Alban Muller, Montreuil/Frankreich unter dem Handelsnamen Amigel® vertriebene Sclerotium Gum. Besonders bevorzugt sind die Copolymere der Acrylsäure oder der Methacrylsäure, wie sie zum Beispiel unter dem Handelsnamen Carbopol 1342 oder Pemulen TR1 der Firma GOODRICH, USA vertrieben werden.

Das erfindungsgemäße Mittel wird bevorzugt in einem wässrigen oder in einem wässrig-alkoholischen Milieu konfektioniert. Desweiteren können Lösungsmittel oder ein Gemisch aus Lösungsmitteln mit einem Siedepunkt unter 400°C in einer Menge von 0,1 bis 90 Gewichtsprozent bevorzugt von 1 bis 50 Gewichtsprozent eingesetzt werden. Besonders geeignet sind unverzweigte oder verzweigte Kohlenwasserstoffe wie Pentan, Hexan, Isopentan und zyklische Kohlenwasserstoffe wie Cyclopentan und Cyclohexan. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Weitere, besonders bevorzugte wasserlösliche Lösungsmittel sind Glycerin und Propylenglykol in einer Menge bis 30 Gewichtsprozent.

Selbstverständlich können die erfindungsgemäßen Mittel auch weitere übliche kosmetische Zusätze, wie beispielsweise nichtfestigende nichtionische Polymere, wie zum Beispiel Polethylenglykole oder Copolymere aus Ethylenglykol und Propylenglykol, nichtfestigende anionische Polymere und nichtfestigende natürliche Polymere sowie deren Kombination in einer Menge von vorzugsweise 0,01 - 50 Gewichtsprozent; Parfümöle in einer Menge von vorzugsweise 0,01 - 5 Gewichtsprozent; Trübungsmittel wie zum Beispiel Ethylenglykoldistearat, in einer Menge von vorzugsweise 0,01 - 5 Gewichtsprozent; Netzmittel oder Emulgatoren aus den Klassen der anionischen, kationischen, amphoteren oder nichtionogenen oberflächenaktiven Substanzen ohne Waschwirkung wie Fettalkoholsulfate, ethoxylierte Fettalkohole, Fettsäurealkanolamide wie zum Beispiel die Ester der hydrierten Rizinusölfettsäuren in einer Menge von vorzugsweise 0,1 - 30 Gewichtsprozent; ferner Feuchthaltemittel, Farbstoffe, Lichtschutzmittel, Antioxidantien, Glanzgeber und Konservierungsstoffe in einer Menge von vorzugsweise 0,01 - 10 Gewichtsprozent enthalten.

Als weitere Zusätze sind geeignete Silikonpolymere zum Beispiel einsetzbar: Polydimethylsiloxan (INCI: Dimethicon), α-Hydro-ω-hydroxypolyoxydimethylsilylen (INCI: Dimethiconol), cyclisches Dimethylpolysiloxan (INCI: Cyclomethicon), Trimethyl(octadecyloxy)silan (INCI: Stearoxytrimethylsilan), Dimethylsiloxan-Glykol-Copolymer (INCI: Dimethicon Copolyol), Dimethylsiloxanaminoalkylsiloxan-Copolymer mit Hydroxyendgruppen (INCI: Amodimethicon), Monomethylpolysiloxan mit Laurylseitenketten und polyoxyethylen- und/oder Polyoxypropylenendketten, (INCI: Laurylmethicon Copolyol), Dimethylsiloxan-Glykol-Copolymer-acetat (INCI: Dimethiconcopolyol Acetat) Dimetylsiloxanaminoalkylsiloxan-Copolymer mit Trimethylsilylendgruppen (INCI: Trimethylsilylamodimethicon). Bevorzugte Silikonpolymere sind: Dimethicone, welche beispielsweise von der Firma Wacker, München/Deutschland unter der Handelsbezeichnung SILOXANE F-221 oder von der Firma Dow Corning Europe, Brüssel/Belgien unter der Handelsbezeichnung Dow Corning Fluid 200/0,65 cs vertrieben werden; Cyclomethicone, die beispielsweise unter den Handelsbezeichnungen DOW CORNING 244 Fluid von der Firma Dow Corning Europe, Brüssel/Belgien oder ABIL® K4 von der Firma Goldschmidt/Deutschland vertrieben werden; Dimethiconole, die beispielsweise unter den Handelsbezeichnungen SILICONE FLUID F-212 von der Firma Wacker/Deutschland oder UNISIL® SF-R von der Firma UPI vertrieben werden.

Die vorstehend in Klammern angegebenen Bezeichnungen entsprechen der INCI Nomenklatur (International Cosmetic Ingredients), wie sie zur Kennzeichnung kosmetischer Wirk- und Hilfsstoffe bestimmt sind.

Auch Mischungen von Silikonpolymeren sind geeignet wie zum Beispiel eine Mischung aus Dimethicon und Dimethiconol, die beispielsweise unter der Handelsbezeichnung DOW CORNING 1403 Fluid von der Firma Dow Corning Europe/Belgien vertrieben wird.

Ein weiterer Vorteil der vorliegenden Erfindung besteht darin, daß fluorierte Säuren, insbesondere perfluorierte Säuren in der Lage sind, Chitosan in wasserhaltigen Aerosolsprayformulierungen wie z.B. Haarsprays, welche einen reduzierten Gehalt an flüchtigen organischen Bestandteilen (volatile organic Compounds, VOC) aufweisen, zu stabilisieren. Gegenstand der vorliegenden Erfindung ist daher auch ein Aerosolspray mit einem Gehalt an

(A) Chitosan oder einem basischen Chitosanderivat,
(B) mindestens einer fluorierten Säure,
(C) Wasser in einer Menge von vorzugsweise mindestens 10, besonders bevorzugt von mindestens 30 Gewichtsprozent und
(D) mindestens einem Treibmittel,
wobei das Spray maximal 80, vorzugsweise maximal 55 Prozent VOC's enthält.

Das erfindungsgemäße Mittel kann in verschiedenen Applikationsformen Anwendung finden, wie beispielsweise in Aerosolzubereitungen als Schaum oder als Spray, des weiteren als Non-Aerosol, welches mittels einer Pumpe oder als „Pump and Spray" zum Einsatz kommt. Der Einsatz in üblichen O/W und W/O Emulsionen ist ebenso möglich wie in Anwendungsformen als Gel, Wachs oder Mikroemulsion. Das erfindungsgemäße Mittel kann auch als färbendes oder pflegendes Haarbehandlungsmittel wie zum Beispiel als Farbfestiger und Haarspülung formuliert sein.

Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt. Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Desweiteren ist mit der erfindungsgemäßen Polymerkombination die Herstellung von Konzentraten möglich, welche einen verringerten Wasser- oder Lösungsmittelgehalt aufweisen. Die Konzentrate werden nach Transport und gegebenenfalls Lagerung durch Zugabe der erforderlichen Menge Wasser oder Lösungsmittel in ein anwendungsfähiges Haarbehandlungsmittel überführt.

Unter Haarbehandlung soll die Behandlung des menschlichen Kopfhaares vor allem zum Zweck der Herstellung einer Frisur oder zur Pflege der Haare verstanden werden.

Die erfindungsgemäßen Mittel weisen ein verbessertes Rückstandsverhalten auf, ohne Festigungsleistung einzubüssen. Zusätzlich wird die Resistenz der Polymerfilme gegenüber Wasserdampfaufnahme erhöht, wodurch eine verbesserte Haltbarkeit der Festigung bei hoher Luftfeuchte erreicht wird.

Die nachfolgenden Beispiele sollen den Gegenstand der Erfindung näher erläutern.

**Beispiele**

**Beispiel 1**

Es wurden folgende Rezepturen erstellt:

|  | Nr. 1 nicht erfindungsgemäß | Nr. 2 erfindungsgemäß |
|---|---|---|
| Chitosan | 0,80 g | 0,80 g |
| Perfluoropentansäure | - | 0,23 g |
| Ameisensäure 85% | 0,24 g | 0,19 g |
| PVP/VA Copolymer | 1,00 g | 1,00 g |
| PVP Polymer | 1,00 g | 1,00 g |
| Polyquaternium 16 | 0,50 g | 0,50 g |
| Cetyltrimethylammonium Chlorid | 0,10 g | 0,10 g |
| Ethanol 94.7% | 10,00 g | 10,00 g |
| Wasser | 86,36 g | 86,18 g |
|  | 100,00 g | 100,00 g |
| Polyquaternium-16: Methylvinylimidazoliumchlorid/Vinylpyrrolidon Copolymer | | |

Die Wirkstofflösung der nicht erfindungsgemäßen Schaumfestiger-Lösung Nr. 1 wurde bezüglich der Rückstandsbildung und der Festigung auf dem Haar mit derjenigen der erfindungsgemäßen Wirkstofflösung Nr. 2 verglichen.

Rückstandsbildung:

Je 5 Haartressen von gleichem Gewicht und gleichen Ausmaßen wurden mit gleicher Menge an Schaumfestigerlösung Hr. 1 und Nr. 2 behandelt und bei 20 °C bei definierter Luftfeuchte getrocknet. Die Messung der Rückstände erfolgte mit Hilfe der Methode der Bildanalyse:

Von jeder Haarsträhne wurde nach einmaligem Durchziehen der Strähne durch einen Metallkamm der Weißanteil von 10 nicht überlappenden Quadraten von 1x1 cm Fläche ermittelt. Die Messungen ergaben folgende Rückstände (in % Weißanteil der Gesamtfläche;

Mittelwert aus 5 Haarsträhnen zu 10 Messquadraten ergibt insgesamt 50 Messungen pro Messwert):

Nr.1  2.69 +/- 0.26
Nr.2  2.04 +/- 0.28

Dies ergibt eine mittlere Erniedrigung der Rückstände in mit Nr. 2 behandelten Haarsträhnen von 24 Prozent gegenüber den mit Nr. 1 behandelten Strähnen. Aus den Resultaten der Meßreihe kann somit eindeutig geschlossen werden, daß durch teilweise Neutralisation des Chitosans mit Perfluorpentansäure die Rückstände vermindert wurden.

Festigungsstärke:

Die Festigungsstärken der Wirkstofflösungen der Schaumfestiger Nr. 1 und Nr. 2 wurden mit Hilfe der Bruchkraftmessung an Haarsträhnen ermittelt (9 Messungen pro Wirkstofflösung, Angaben in Newton).

Nr.1  0.123 +/- 0.029

Nr.2     0.131 +/- 0.025

Aus dieser Messreihe folgt, daß keine signifikante Veränderung der Festigungsleistung zwischen Nr. 1 und Nr. 2 feststellbar ist.

Aus der Kombination der Rückstands- und der Bruchkraftmessungen folgt, daß die Verwendung der perfluorierten Säure in Nr. 2 bei Beihaltung der Festigungsstärke zu einer Verringerung der Rückstände geführt hat.

**Beispiel 2: Schaumfestiger**

| | |
|---|---|
| 1,00 g | Polyvinylpyrrolidon |
| 1,00 g | PVP/Vinylacetat Copolymer |
| 1,00 g | Chitosan |
| 0,24 g | Ameisensäure, 85 %ig |
| 0,29 g | Perfluorpentansäure |
| 0,20 g | Cetyltrimethylammoniumchlorid |
| 1,00 g | Polyquaternium 16 |
| 10,00 g | Ethanol |
| <u>85,27 g</u> | Wasser |
| 100,00 g | |

Die Abfüllung erfolgt mit 92% Wirkstofflösung und 8% Propan/Butan. Der Schaum ergibt eine gute, geschmeidige Festigung und läßt sich durch Ausbürsten gut wieder entfernen, ohne daß die Haare belastet werden.

**Beispiel 3: Schaumfestiger mit starker Festigung**

| | |
|---|---|
| 0,50 g | Polyvinylpyrrolidon |
| 0,50 g | PVP/Vinylacetat Copolymer |
| 0,50 g | hochmolekulares Chitosan, 300.000-700.000 g/mol |
| 0,35 g | Pyrrolidoncarbonsäure |
| 0,17 g | Pentafluorpropionsäure |
| 0,20 g | Cetyltrimethylammoniumchlorid |
| 1,00 g | Polyquaternium 16 |
| 10,00 g | Ethanol |
| <u>86,78 g</u> | Wasser |
| 100,00 g | |

Die Abfüllung erfolgt mit 92% Wirkstofflösung und 8% Propan/Butan. Der Schaum ergibt eine gute, geschmeidige Festigung und läßt sich durch Ausbürsten gut wieder entfernen, ohne daß die Haare belastet werden.

**Beispiel 4: Schaumfestiger**

| | |
|---|---|
| 1,00 g | Polyvinylpyrrolidon |
| 1,00 g | PVP/Vinylacetat Copolymer |
| 1,00 g | Chitosan |
| 0,24 g | Ameisensäure, 85 %ig |
| 0,24 g | Perfluorbuttersäure |
| 0,20 g | Cetyltrimethylammoniumchlorid |
| 1,00 g | Polyquaternium 16 |
| 10,00 g | Ethanol |
| <u>85,32 g</u> | Wasser |
| 100,00 g | |

Die Abfüllung erfolgt mit 92% Wirkstofflösung und 8% Propan/Butan. Der Schaum ergibt eine gute, geschmeidige Festigung und läßt sich durch Ausbürsten gut wieder entfernen, ohne daß die Haare belastet werden.

**Beispiel 5: Schaumfestiger mit starker Festigung**

| | |
|---|---|
| 0,50 g | Polyvinylpyrrolidon |
| 0,50 g | PVP/Vinylacetat Copolymer |
| 0,50 g | hochmolekulares Chitosan (MW = 300.000 - 700.000 g/mol) |
| 0,35 g | Pyrrolidoncarbonsäure |
| 0,15 g | Perfluorbuttersäure |
| 0,20 g | Cetyltrimethylammoniumchlorid |
| 1,00 g | Polyquaternium 16 |
| 10,00 g | Ethanol |
| <u>86,80 g</u> | Wasser |
| 100,00 g | |

Die Abfüllung erfolgt mit 92% Wirkstofflösung und 8% Propan/Butan. Der Schaum ergibt eine gute, geschmeidige Festigung und läßt sich durch Ausbürsten gut wieder entfernen, ohne daß die Haare belastet werden.

**Beispiel 6: Schaumfestiger ohne Ethanol**

| | |
|---|---|
| 1,00 g | Polyvinylpyrrolidon |
| 1,00 g | PVP/Vinylacetat Copolymer |
| 1,00 g | Chitosan |
| 0,24 g | Ameisensäure, 85 %ig |
| 0,29 g | Perfluorpentansäure |
| 0,20 g | Cetyltrimethylammoniumchlorid |
| 1,00 g | Polyquaternium 16 |
| <u>95,27 g</u> | Wasser |
| 100,00 g | |

Die Abfüllung erfolgt mit 92% Wirkstofflösung und 8% Propan/Butan. Der Schaum ergibt eine gute, geschmeidige Festigung und läßt sich durch Ausbürsten gut wieder entfernen, ohne daß die Haare belastet werden.

**Beispiel 7: Schaumfestiger mit erhöhtem Glanz**

| | |
|---|---|
| 1,00 g | Polyvinylpyrrolidon |
| 1,00 g | PVP/Vinylacetat Copolymer |
| 1,00 g | Chitosan |
| 0,24 g | Ameisensäure, 85 %ig |
| 0,29 g | Perfluorcaprylsäure |
| 0,20 g | Cetyltrimethylammoniumchlorid |
| 1,00 g | Polyquaternium 16 |
| 0,10 g | ethoxylierter Fettalkohol |
| 0,20 g | Polydimethylsiloxan |
| 20,00 g | Ethanol |
| <u>74,97 g</u> | Wasser |
| 100,00 g | |

Die Abfüllung erfolgt mit 92% Wirkstofflösung und 8% Propan/Butan.

**Beispiel 8: Flüssigfestiger**

| | |
|---|---|
| 1,00 g | Vinylacetat/Crotonsäure Copolymer, 60 %ige Lösung in Isopropanol/Wasser (ARISTOFLEX®) |
| 0,70 g | Amphomer |
| 0,15 g | Aminomethylpropanol |
| 0,20 | g Mischung aus perfluorierten Phosphin-/ Phosphonsäuren (Fluowet® PL 80) |
| 0,10 g | Glycerin |
| 50,00 g | Ethanol |
| 0,50 g | Parfümöl |
| 47,35 g | Wasser |
| 100,00 g | |

Amphomer und Aristoflex werden in Ethanol gelöst. Dann wird Aminomethylpropanol zugeben und 1 h bei Raumtemperatur gerührt. Anschließend werden die übrigen Bestandteile zugeben, zum Schluß das Wasser.

**Beispiel 9: Gelförmiger Festiger**

| | |
|---|---|
| 1,00 g | Chitosan |
| 0,15 g | Ameisensäure 85% |
| 0,45 g | Pentafluorpropionsäure |
| 3,00 g | Vinylpyrrolidon-Vinylacetat-Copolymer |
| 1,00 g | Hydroxyethylcellulose |
| 0,10 g | Glycerin |
| 94,30 g | Wasser |
| 100,00 g | |

**Beispiel 10: Gelförmiger Festiger mit starker Festigung**

| | |
|---|---|
| 1,00 g | hochmolekulares Chitosan (MW = 300.000 - 700.000 g/mol) |
| 0,70 g | Pyrrolidoncarbonsäure |
| 0,34 g | Perfluorpentansäure |
| 3,00 g | Vinylpyrrolidon-Vinylacetat-Copolymer |
| 1,00 g | Hydroxyethylcellulose |
| 0,10 g | Propylenglykol |
| 30,00 g | Ethanol |
| 63,86 g | Wasser |
| 100,00 g | |

**Beispiel 11: Gelförmiger Festiger mit starker Festigung**

| | |
|---|---|
| 1,00 g | hochmolekulares Chitosan (MW = 300.000 - 700.000 g/mol) |
| 0,44 g | Pyrrolidoncarbonsäure |
| 0,53 g | Perfluorpropionsäure |
| 3,00 g | Vinylpyrrolidon-Vinylacetat-Copolymer |
| 1,00 g | Hydroxyethylcellulose |
| 0,10 g | Propylenglykol |
| 30,00 g | Ethanol |
| 63,86 g | Wasser |
| 100,00 g | |

**Beispiel 12: Non-Aerosol Haarspray**

| | |
|---|---|
| 0,20 g | Perfluoroctansäure (Fluorad® FX-1001) |
| 0,20 g | Aminomethylpropanol |
| 1,00 g | Amphomer |
| 3,00 g | Vinylpyrrolidon-Vinylacetat-Copolymer |
| 50,00 g | Ethanol |
| 45,60 g | Wasser |
| 100,00 g | |

Amphomer und Aminomethylpropanol werden in Ethanol gelöst. Anschließend wird Fluorad® FX-1001 und dann Vinylpyrrolidon-Vinylacetat-Copolymer zugegeben und gelöst. Zum Schluß wird das Wasser zugefügt.

**Beispiel 13: Aerosol Haarspray**

| | |
|---|---|
| 0,10 g | Perfluoroctansulfonsäure |
| 0,40 g | Aminomethylpropanol |
| 2,00 g | Amphomer |
| 3,00 g | Vinylpyrrolidon-Vinylacetat-Copolymer |
| 50,00 g | Ethanol |
| 44,70 g | Propan/Butan |
| 100,00 g | |

Aminomethylpropanol wird in Ethanol vorgelegt und Amphomer darin gelöst. Die restlichen Bestandteile werden zugegeben und gelöst. Anschließend wird mit Propan/Butan befüllt.

**Beispiel 14: Haarfestiger**

| | |
|---|---|
| 1,00 g | Chitosan |
| 0,24 g | Ameisensäure, 85 %ig |
| 0,29 g | Perfluorpentansäure |
| 10,00 g | Ethanol |
| 88,47 g | Wasser |
| 100,00 g | |

**Beispiel 15: Sprays mit reduziertem Gehalt an VOC's**

Es wurden VOC 55-Sprays und VOC 80-Sprays mit einem Gehalt an Chitosan in Abhängigkeit von den zur Neutralisation des Chitosans verwendeten Säuren untersucht.

VOC 55-Rezeptur:

| | |
|---|---|
| Chitosan | 4,0 g |
| Neutralisationsmittel (s. Tabelle 1) | die zur vollständigen Neutralisation von Chitosan benötigte Menge |
| Ethanol | 20 g |

(fortgesetzt)

| | |
|---|---|
| Dimethylether | 35 g |
| Wasser | ad 100 g |

VOC 80-Rezeptur:

| | |
|---|---|
| Chitosan | 4,0 g |
| Neutralisationsmittel (s. Tabelle 1) | die zur vollständigen Neutralisation von Chitosan benötigte Menge |
| Ethanol | 40 g |
| Dimethylether | 35 g |
| Wasser | ad 100 g |

Die Ergebnisse sind in der folgenden Tabelle 1 dargestellt:

| Neutralisationsmittel | Ergebnis VOC 80-Rezeptur | Ergebnis VOC 55-Rezeptur |
|---|---|---|
| Ameisensäure | Ausfällung des Chitosans | Ausfällung des Chitosans |
| Pyrrolidoncarbonsäure | Ausfällung des Chitosans | zweiphasig, schnelle Auftrennung |
| Milchsäure | Ausfällung des Chitosans | zweiphasig, schnelle Auftrennung |
| 3,6,9-Trioxaundecansäure | Ausfällung des Chitosans | Ausfällung des Chitosans |
| Perfluorpentansäure | einphasig, klar | einphasig, klar |

Nur bei Verwendung der fluorierten Säure perfluorpentansäure entstand eine ohne vorheriges Schütteln versprühbare, einphasige, niedrigviskose Rezeptur.

**Patentansprüche**

1. Verwendung von fluorierten Säuren zur Neutralisation von Polymeren mit basischen Gruppen in Mitteln zur Behandlung von Haaren.

2. Verwendung nach Anspruch 1 in Mitteln zur Festigung von Haaren.

3. Haarbehandlungsmittel mit einem Gehalt an einem Komplex gebildet aus mindestens einer fluorierten Säure und mindestens einem Polymeren mit primären oder sekundären Amingruppen bzw. einem Komplex eines Salzes einer fluorierten Säure und einem Polymeren mit protonierten primären oder sekundären Amingruppen.

4. Haarbehandlungsmittel mit einem Gehalt an

   (A) einem Komplex gebildet aus mindestens einer fluorierten Säure und mindestens einem Polymeren mit basischen Gruppen bzw. einem Komplex eines Salzes einer fluorierten Säure und einem Polymeren mit protonierten oder quaternären Amingruppen und
   (B) zusätzlich mindestens einem filmbildenden, haarfestigenden Polymeren.

5. Mittel nach Anspruch 3 oder 4, dadurch gekennzeichnet, daß es 0,01 bis 10 Gewichtsprozent der fluorierten Säure bzw. dessen Salzes enthält.

**6.** Mittel nach einem der Ansprüche 3 bis 5, dadurch gekennzeichnet, daß die fluorierte Säure eine Verbindung ist mit der allgemeinen Formel

$$A-(CH_2)_x-(CF_2)_y-(CH_2)_z-B$$

wobei x und z unabhängig voneinander Zahlen von 0 bis 5 bedeuten, y eine Zahl von 1 bis 21 bedeutet, A eine Säuregruppe ist und B entweder für eine Säuregruppe oder für ein Fluoratom steht, wobei z den Wert 0 hat, wenn B für ein Fluoratom steht.

**7.** Mittel nach Anspruch 6, dadurch gekennzeichnet, daß die Säuregruppen A- und B- ausgewählt sind aus -COOH, -SO$_3$H, -OPO$_2$H und -OPO$_3$H$_2$.

**8.** Mittel nach einem der Ansprüche 3 bis 7, dadurch gekennzeichnet, daß es 0,01 bis 20 Gewichtsprozenr des Polymers mit basischen Gruppen bzw. des Polymers mit protonierten Amingruppen enthält.

**9.** Mittel nach einem der Ansprüche 3 bis 8, dadurch gekennzeichnet, daß das Polymer mit basischen Gruppen in nicht neutralisierter Form wasserunlöslich ist.

**10.** Mittel nach einem der Ansprüche 3 bis 9, dadurch gekennzeichnet, daß das Polymer mit basischen Gruppen Chitosan ist.

**11.** Mittel nach einem der Ansprüche 3 bis 9, dadurch gekennzeichnet, daß das Polymer mit basischen Gruppen ein amphoteres Polymer ist.

**12.** Mittel nach Anspruch 11, dadurch gekennzeichnet, daß saure Gruppen des amphoteren Polymers mit einem basischen Neutralisationsmittel neutralisiert sind.

**13.** Mittel nach einem der Ansprüche 3 bis 12, dadurch gekennzeichnet, daß die basischen Gruppen des Polymers zu 5 bis 70 Prozent mit der fluorierten Säure neutralisiert sind.

**14.** Mittel nach Anspruch 13, dadurch gekennzeichnet, daß die basischen Gruppen zusätzlich mit mindestens einer weiteren, nicht fluorierten Säure neutralisiert sind.

**15.** Mittel nach einem der Ansprüche 4 bis 14, dadurch gekennzeichnet, daß das filmbildende, haarfestigende Polymer (B) in einer Menge von 0,01 bis 50 Gewichtsprozent enthalten ist.

**16.** Aerosolspray mit einem Gehalt an

    (A) Chitosan oder einem basischen Chitosanderivat,
    (B) mindestens einer fluorierten Säure,
    (C) Wasser und
    (D) mindestens einem Treibmittel,
    wobei das Spray maximal 80 Prozent VOC's enthält.